# EUROPÄISCHE PATENTSCHRIFT

(11) **EP 2 773 347 B1**
(45) Veröffentlichungstag und Bekanntmachung des Hinweises auf die Patenterteilung: **13.09.2017**
(21) Anmeldenummer: 12733105.6
(22) Anmeldetag: 05.07.2012
(51) Int. Cl.: A61K 31/4439, A61P 7/02, C07D 401/12

(54) **DABIGATRAN-AMIDOXIMSÄUREESTER ALS PRODRUGS UND IHRE VERWENDUNG ALS ARZNEIMITTEL**
AMIDOXIME CARBOXYLIC ACID ESTERS OF DABIGATRAN AS PRODRUGS AND THEIR USE AS MEDICAMENTS
ESTERS D'ACIDE CARBOXYLIQUE D'AMIDOXIME DU DABIGATRAN EN TANT QUE PRODROGUES ET LEUR UTILISATION COMME MÉDICAMENT

(30) Priorität: 25.07.2011 EP 11175230
(43) Veröffentlichungstag der Anmeldung: 10.09.2014
(73) Patentinhaber: Develco Pharma Schweiz AG, 4133 Pratteln (CH)
(72) Erfinder: CLEMENT, Bernd, 24106 Kiel (DE); KOTTHAUS, Jürke, 24118 Kiel (DE); KOTTHAUS, Joscha, 24116 Kiel (DE); SCHADE, Dennis, La Jolla, California 92037 (US)
(74) Vertreter: Ter Meer Steinmeister & Partner
(86) Internationale Anmeldenummer: PCT/EP2012/063071
(87) Internationale Veröffentlichungsnummer: WO 2013/013946

(56) Entgegenhaltungen:
- WO-A1-2004/014894
- WO-A1-2010/055022
- US-A1- 2011 028 756

## Beschreibung

Die vorliegende Erfindung betrifft Prodrug-Derivate des Dabigatrans, ihre Verwendung zur Behandlung und/oder Prophylaxe von Krankheiten, insbesondere von thrombotischen Erkrankungen, von Schlaganfall, Herzinfarkt und/oder Vorhofflimmern und Arrhytmien, sowie von onkologischen Erkrankungen jeglicher Pathogenese.

Innerhalb der letzten Jahre hat sich Dabigatran Etexilat (Pradaxa®) in der Thrombose-Prophylaxe nach Hüft- und Kniegelenks-Operationen etabliert.¹ Zusätzlich wurde dieser Wirkstoff für weitere Indikationsgebiete (Vorhofflimmern, Schlaganfall-Prophylaxe und Sekundärprophylaxe nach Herzinfarkt, akutes Koronar-Syndrom zugelassen.^{2,3} Zudem sind auf lange Sicht weitere aussichtsreiche Indikationsgebiete denkbar, besonders im cardiovasculären Bereich sowie zur Krebs-Therapie.⁴ Der erfolgreichen Markteinführung zum Trotz besitzt das Dabigatran-Etexilat auch ungünstige Stoffeigenschaften, die seine breite Verwendung einschränken können.

So ist das Dabigatran-Etexilat also Prodrug des eigentlichen Wirkstoffes Dabigatran beispielsweise sehr schlecht löslich, was zu einigen Nachteilen sowohl in der Anwendung als auch in der Herstellung des Arzneimittels führt. Um die Löslichkeit der Verbindung zu verbessern wird der Arzneistoff auf Pellets, die Weinsäure enthalten, aufgetragen, was aufgrund eines erheblichen technischen Aufwands zur Herstellung dieser galenischen Form sehr kostenintensiv ist.⁵⁻⁷ Außerdem wird die Bioverfügbarkeit durch die schlechte Löslichkeit der Verbindung negativ beeinflusst, so dass die Medikation aus zwei Kapseln besteht, was wiederum die Compliance der Patienten negativ beeinflusst.

Ziel der vorliegenden Erfindung war es Prodrugs des Dabigatrans zu entwickeln, die neben einer ausreichenden oralen Bioverfügbarkeit auch über verbesserte Stoffeigenschaften, wie beispielsweise verbesserte Löslichkeit verfügen. Zu diesem Zweck wurden verschiedene Dabigatran-Derivate synthetisiert. Durch die *N*-Hydroxylierung der Amidin-Funktion des Dabigatrans wurde das Dabigatran in das Dabigatran-Amidoxim (**2**) überführt, wodurch eine Reduzierung der Basizität und eine gesteigerte Absorption aus dem Magen-Darm-Trakt resultiert.⁸ Innerhalb unserer Studien wurde zudem Bezug auf das Prodrug-Prinzip "Kopplung von Amidoximen mit Dicarbonsäuren", wie es in den Patentanmeldungen [WO2009095499, DE102008007381] beschrieben ist, genommen.⁹ Dieses Prodrug-Prinzip wurde auf das Dabigatran übertragen, die erhaltenen Verbindungen wurde ausführlich charakterisiert und hinsichtlich ihrer Bioverfügbarkeit untersucht.

Bei dem Wirkstoff Dabigatran handelt es sich um einen hochpotenten Thrombin-Inhibitor, der oral nicht verfügbar ist. Aus diesem Grund wird die Verbindung derzeit als Etexilat-Prodrug (Dabigatran-Etexilat, Pradaxa®) verwendet. Obwohl die Verbindung oral verfügbar und gut wirksam ist, besitzt die Verbindung aufgrund des verwendeten Prodrug-Prinzips erhebliche negative Eigenschaften. Die sehr schlechte Löslichkeit führt zu einigen Nachteilen sowohl bei der Anwendung als auch bei der Herstellung des Arzneimittels. Um die Löslichkeit der Verbindung zu verbessern wird der Arzneistoff auf Pellets, die Weinsäure enthalten, aufgetragen, was aufgrund eines erheblichen technischen Aufwands sehr kostenintensiv ist.^{5,7,12} Außerdem wird die Bioverfügbarkeit durch die schlechte Löslichkeit der Verbindung negativ beeinflusst, so dass die Medikation aus zwei Kapseln besteht, was wiederum die Compliance negativ beeinflusst. Vor diesem Hintergrund lag der vorliegenden Erfindung die Aufgabe zugrunde, Dabigatran-Prodrugs bereitzustellen, die gegenüber den bekannten Darreichungsformen von Dabigatran verbesserte Eigenschaften aufweisen.

Die genannte Aufgabe wird erfindungsgemäß gelöst durch eine Verbindung der Formel (I) 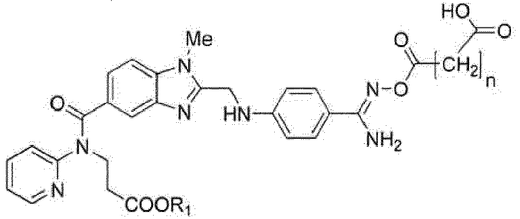 in welcher
- R¹: für ein -H, eine verzweigte oder unverzweigte, gesättigte oder ungesättigte, nicht substituierte Kohlenwasserstoffkette mit einer Kettenlänge von 1 bis 12 steht und
- n: für 1-10 steht.

In einer bevorzugten Ausführungsform steht n in Formel (I) für 2.

In einer weiteren bevorzugten Ausführungsform steht R¹ in Formel (I) für Ethyl.

In einer besonders bevorzugten Ausführungsform handelt es sich bei der erfindungsgemäßen Verbindung um Dabigatran-Amidoxim-Bernsteinsäureester (**1**). In vergleichenden Studien mit anderen Dabigatran-Prodrugs stellte sich der Dabigatran-Amidoxim-Bernsteinsäureester (**1**) als vorteilhaftes Dabigatran-Prodrug heraus. Die Verbindung besitzt eine ausgezeichnete Löslichkeit, eine geeignete Stabilität und eine gute orale Bioverfügbarkeit. Außerdem wird das Prodrug leicht in die aktive Form Dabigatran konvertiert. Die Aktivierung verläuft über Esterasen sowie ein Molybdän-haltiges Enzymsystem (mARC) und ist damit unabhängig von Cytochrom P450 Enzymen, welche das Risiko von Interaktionen besitzen würden.^{8,10,11}

Die vorliegende Erfindung betrifft darüber hinaus auch Salze, Solvate und Solvate der Salze der genannten Verbindungen der Formel (I).

Ferner betrifft die vorliegende Erfindung die genannten Verbindungen der Formel (I) zur Behandlung und/oder Prophylaxe von Krankheiten.

In einer bevorzugten Ausführungsform betrifft die vorliegende Erfindung die genannten Verbindungen zur Verwendung bei der Behandlung und/oder Prophylaxe von thrombotischen Erkrankungen.

In einer weiteren bevorzugten Ausführungsform betrifft die vorliegende Erfindung die genannten Verbindungen der Formel (I) zur Verwendung bei der Behandlung und/oder Prophylaxe von thrombotischen Ereignissen, insbesondere der venösen Thromboembolie (VTE).

In einer weiteren bevorzugten Ausführungsform betrifft die vorliegende Erfindung die genannten Verbindungen der Formel (I) zur Verwendung bei der Behandlung und/oder Prophylaxe von Schlaganfall, Herzinfarkt und/oder Vorhofflimmern und Arrhytmien.

In einer weiteren bevorzugten Ausführungsform betrifft die vorliegende Erfindung die genannten Verbindungen der Formel (I) zur Verwendung bei der Behandlung und/oder Prophylaxe onkologischen Erkrankungen jeglicher Pathogenese.

Die vorliegende Erfindung betrifft auch ein Arzneimittel umfassend mindestens eine der genannten Verbindungen der Formel (I) mit einer die Thrombinzeit verlängernden Wirkung, einer thrombinhemmenden Wirkung und/oder einer Hemmwirkung auf verwandte Serinproteasen.

Ferner betrifft die voliegende Erfindung auch ein Arzneimittel umfassend mindestens eine der genannten Verbindungen der Formel (I) gegebenenfalls in Kombination mit einem inerten, nicht-toxischen, pharmazeutisch geeignetem Hilfsstoff.

Die vorliegende Erfindung betrifft darüber hinaus auch ein Arzneimittel umfassend mindestens eine der genannten Verbindungen der Formel (I) in Kombination mit einem weiteren Wirkstoff.

Die vorliegende Erfindung betrifft darüber hinaus auch ein Arzneimittel zur oralen oder parenteralen Anwendung.

Ferner betrifft die vorliegende Erfindung auch ein Arzneimittel wie oben beschrieben, das magensaftresistent formuliert ist.

Die vorliegende Erfindung betrifft auch ein Verfahren zur Herstellung der erfindungsgemäßen Verbindungen gemäß einem der Ansprüche 1 bis 4, dadurch gekennzeichnet, dass ein Nitril der Formel (A) 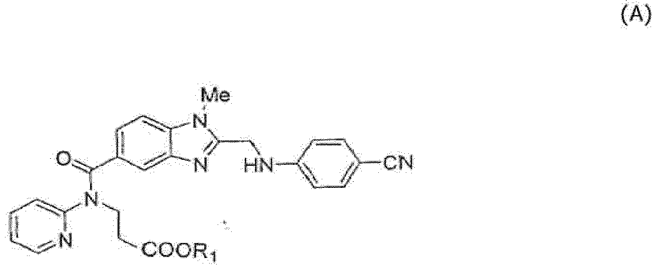 in welcher R¹ für ein -H, eine verzweigte oder unverzweigte, gesättigte oder ungesättigte, nicht substituierte Kohlenwasserstoffkette mit einer Kettenlänge von 1 bis 12 steht
zu einem Amidoxim der Formel (B) umgesetzt wird 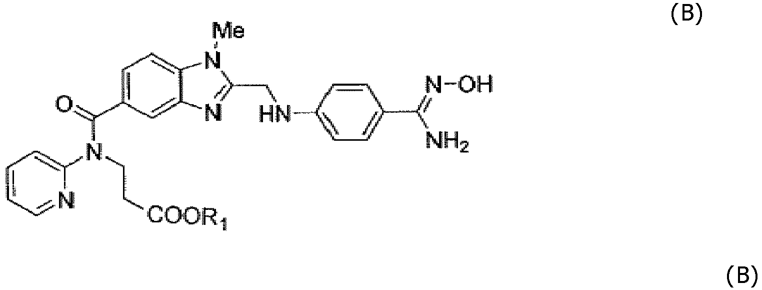 und das so gewonnene Amidoxim durch Reaktion mit einem Dicarbonsäureanhydrid der Formel (C) 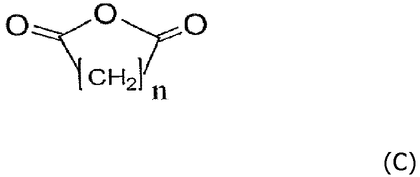 in welcher n für 1-10 steht
in eine Verbindung der Formel 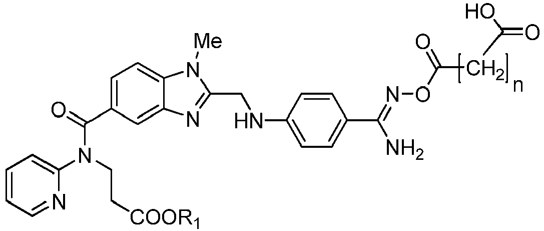 überführt wird. Die vorliegende Erfindung befasst sich mit der Entwicklung neuer Dabigatran-Prodrugs mit gegenüber dem Dabigatran-Etexilat verbesserten Eigenschaften. Innerhalb unserer systematischen Entwicklung und anschließender Charakterisierung der neuen Prodrugs, hat sich der Dabigatran-Amidoxim-Bernsteinsäureester (**1**) als hervorragend geeignetes Prodrug erwiesen. Das Prodrug zeichnet sich durch exzellente Eigenschaften, wie gute Löslichkeit, schnelle Aktivierung und eine dem Dabigatran-Etexilat vergleichbare orale Bioverfügbarkeit aus. Der entscheidende Vorteil dieses Prodrugs liegt in seinen verbesserten Stoffeigenschaften: Aufgrund der erheblich gesteigerten Löslichkeit kann auf die aufwendige pharmazeutische Formulierung, die beim Pradaxa® erforderlich ist, verzichtet werden, was unter anderem zu einer erhebliche Reduzierung der Herstellungskosten führt. Außerdem kann bei einer anderen galenischen Formulierung die erforderliche Wirkstoff-Dosis des Dabigatran-Amidoxim-Bernsteinsäureesters (**1**) in einer Tablette bzw. Kapsel oral verabreicht werden, was zu einer erheblichen Verbesserung der Compliance der Patienten führen kann. Zudem sind aufgrund der guten Löslichkeit des Prodrugs auch parenterale Applikationsformen (Injektionen, Infusionen, etc.) der Verbindung denkbar, die bei der Verwendung des Pradaxa® nicht möglich sind.

Ein weiterer Aspekt, der durch das hier beschriebene Prodrug verbessert werden könnte, ist die Reduzierung der beim Dabigatran-Etexilat beschriebenen Nebenwirkungen, insbesondere das beschriebene Auftreten von Magen-DarmBlutungen

Zusammengefasst konnte durch die Anwendung des allgemeinen Prodrug-Prinzips [WO2009095499, DE102008007381] ein Prodrug des Dabigatrans erhalten werden, dass einen erhebliche Verbesserung gegenüber dem bisher bekannten Arzneistoffs Dabigatran-Etexilat darstellt. Durch die Verwendung des Dabigatran-Amidoxim-Bernsteinsäureesters (**1**) können zum einem die Herstellungskosten drastisch reduziert werden, zum anderen die klinische Anwendung entscheidend optimiert werden.

### Synthese

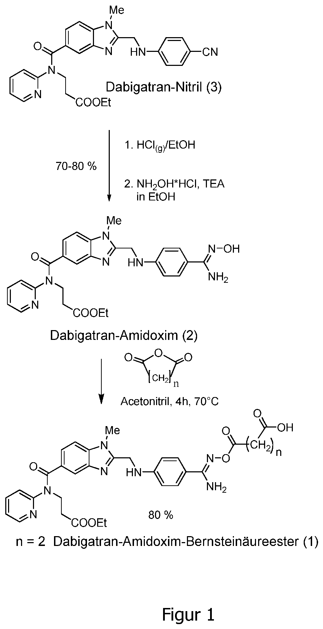

Die Darstellung des Dabigatran-Amidoxim-Bernsteinsäureesters (**1**) erfolgte ausgehend vom Dabigatran-Nitril (**3**) über das Dabigatran-Amidoxim (**2**) gemäß Abbildung 1. Das Dabigatran-Amidoxim (**2**) wird in getrocknetem MeCN suspendiert und mit dem jeweiligen Säureanhydrid (Bernsteinsäure-Anhydrid, etc) zum entsprechenden Ester umgesetzt. Durch anschließende Zugabe von Diethylether und direktes Abfiltrieren konnte die Substanz isoliert werden.

### Stabilität

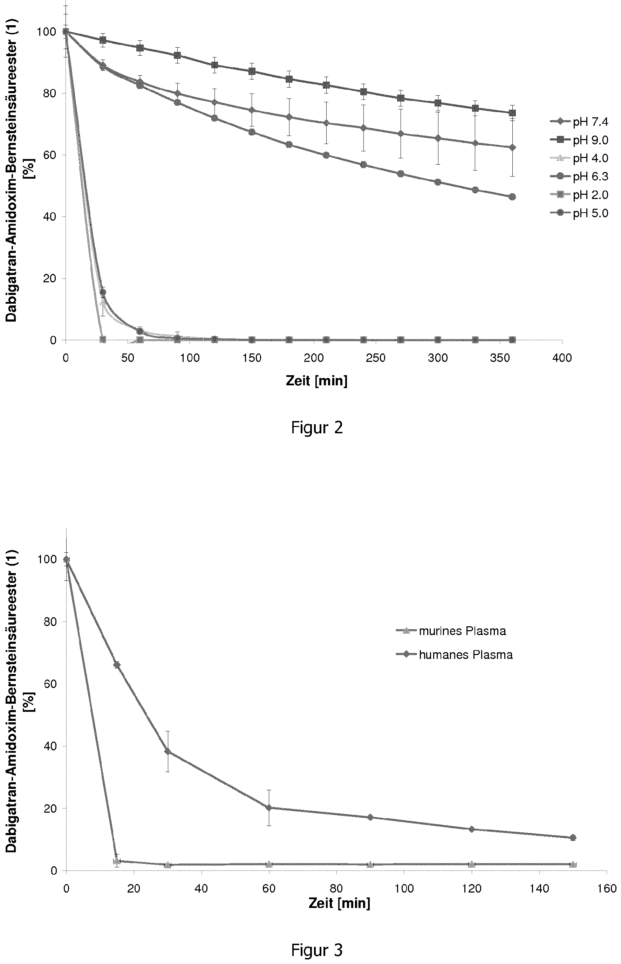

Bei den Untersuchungen der Stabilität konnte gezeigt werden, dass der Dabigatran-Amidoxim-Bernsteinsäureester (**1**) im sauren Milieu (< pH 6) recht instabil ist (Abbildung 2). Die Succinylester-Bindung wird vollständig gespalten, so dass das Dabigatran-Amidoxim (**2**) entsteht. Im neutralen bzw. leicht alkalischen pH-Bereich ist die Verbindung deutlich stabiler. In dem untersuchten Zeitraum von 360 min wurde bei einem pH-Wert von 9.0 eine Spaltung des Succinylester von ca 25% und bei einem pH-Wert von 7.4 von etwa 40% ermittelt werden. Aus diesem Daten folgt, dass die Verbindung für einen späteren Einsatz als Arzneistoff magensaftresistent formuliert werden sollte, damit sie die MagenPassage unbeschadet übersteht und somit vollständig in den oberen Darmabschnitten resorbiert werden kann.

Die Inkubationen in humanem und murinem Plasma zeigten erwartungsgemäß eine ausgeprägte Hydrolyse der Esterbindung (Abbildung 3). Diese Hydrolyse im Plasma ist erwünscht, da sie zur Aktivierung des Prodrugs und somit Freisetzung des Wirkstoffes Dabigatran führt. Sie wird durch Esterasen katalysiert, welche im Plasma ubiquitär vorhanden sind.

### Löslichkeit

Der Dabigatran-Amidoxim-Bernsteinsäureester (**1**) besitzt eine sehr gute Löslichkeit im untersuchten pH Bereich von 6.3 bis 9.0 (s. Tabelle 1). Die Löslichkeit im sauren Milieu (pH 2.0) konnte aufgrund der zuvor beschriebenen Hydrolyse in diesem Milieu nicht exakt charakterisiert werden. Vorversuche zeigten allerdings, dass auch hier die Löslichkeit gut ist.

Tabelle 1 zeigt die Löslichkeit des Dabigatran-Amidoxim-Bernsteinsäureesters (**1**) im Vergleich zu anderen Dabigatran-Prodrugs. Besonders hervorzuheben ist hier der Vergleich zum Dabigatran-Etexilat. Aus den erhaltenen Daten wird deutlich, dass die Löslichkeit des neu entwickelten Dabigatran-Prodrugs (**1**) drastisch verbessert wurde. So ist die Löslichkeit im Vergleich zum Etexilat-Prodrug je nach pH-Wert zwischen 1000 und 100.000fach verbessert, was seine Verwendung Arzneimitteln begünstigt. Zusätzlich sind durch die gute Löslichkeit des Dabigatran-Amidoxim-Bernsteinsäureesters (**1**) auch parenterale Applikationsformen denkbar, wie beispielsweise Injektionen oder Infusionen.

### Proteinbindung

Die Untersuchungen zur Plasmaproteinbindung zeigten, dass die Verbindung **(1)** mit einer Plasmaproteinbindung von ca. 22% eine sehr gering ausgeprägte Proteinbindung zeigt. Proteinbindungen sind erst ab einem Wett von ca. 90% als kritisch hinsichtlich ihres Interaktions-Potenzials einzustufen.¹³ Somit ist der Dabigatran-Amidoxim-Bernsteinsäureester (**1**) in dieser Hinsicht als unkritisch einzustufen.

### Prodrug-Konzept

Das Prodrug-Konzept selbst wurde im Patent [WO2009095499, DE102008007381] anhand anderer Ausführungsbeispiele beschreiben. Das Konzept wurde in dieser Arbeit auf das Dabigatran übertragen. Diese neu entwickelte Verbindung Dabigatran-Amidoxim-Bernsteinsäureester **(1)** hat sich nun - nach eingehender Charakterisierung sowohl in *in vitro als* auch in *in vivo* Studien - als sehr geeignetes Dabigatran-Prodrug zur Entwicklung von Arzneimitteln erwiesen.

Die Aktivierung des Prodrugs verläuft über Esterasen und das mARC-Enzymsystem und ist somit unabhängig von Cytochrom P450-Enzymen.^{8, 10, 11} Die Beteiligung von P450 Enzymen birgt stets das Risiko von Interaktionen, welche bei dem von uns gewählten Aktivierungsmechanismus nicht beschrieben sind.

### In vitro Aktivierung

Die durchgeführten *in vitro* Aktivierungsstudien zeigten, dass die Aktivierung des Dabigatran-Amidoxim-Bernsteinsäureesters (**1**) in einem exzellenten Ausmaß erfolgt (Tabelle 2).

Bereits die Inkubationen in humanem und murinem Plasma zeigten, dass die Esterspaltung sehr ausgeprägt verläuft, was zum Aktivieren des Prodrugs notwendig ist (Abbildung 3).

Bei den Inkubationen mit subzellulären Enzympräparationen konnte auch die anschließende Reduktion zum Dabigatran nachgewiesen werden (Tabelle 2). Die ermittelten Umsetzungsraten bei den Inkubationen mit porcinen Enzymquellen zeigten, dass der Dabigatran-Amidoxim-Bernsteinsäureesters (**1**) ausgezeichnet in die Wirkform umgewandelt wird. Erwartungsgemäß erfolgte die Reduktion vom Amidoxim zum Amidin bei den Mikrosomen und Mitochondrien-Präparationen schneller als bei den 9000xg Fraktionen.

Zusammenfassend kann festgehalten werden, dass es sich bei dem Dabigatran-Amidoxim-Bernsteinsäureester (**1**) um ein sehr geeignetes Prodrug des Dabigatrans handelt. Sowohl die Esterspaltung als auch die Reduktion laufen in einem Ausmaß ab, dass sich therapeutisch wirksame Plasmaspiegel an Dabigatran bilden können.

### Orale Bioverfügbarkeit

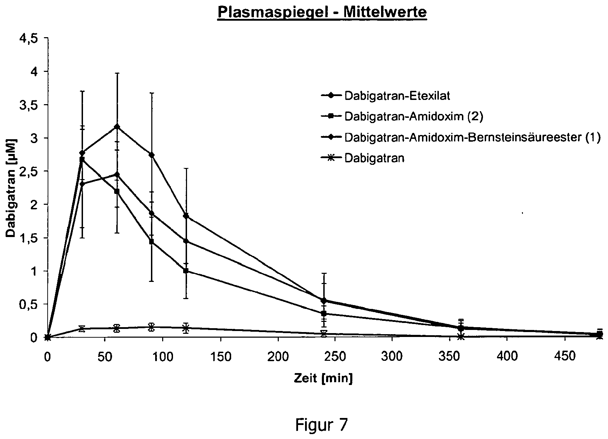

In den durchgeführten Tierstudien konnte die orale Bioverfügbarkeit des Dabigatran-Amidoxim-Bernsteinsäureesters (**1**) demonstriert werden. Nach oraler Applikation des Prodrugs konnten Dabigatran-Plasmaspiegel über einen Zeitraum von 480 min gemessen werden, die den nach oraler Applikation des Dabigatran-Etexilates vergleichbar sind (Abbildung 7, Tabelle 3). Neben der Wirkform Dabigatran konnten keine weiteren Metabolite nachgewiesen werden, was für eine rasche und vollständige Aktivierung der Prodrugs spricht. Die orale Bioverfügbarkeit des Dabigatran-Amidoxim-Bernsteinsäureesters (**1**) wurde mit 5.5% ± 1.7% ermittelt. Die maximalen Plasmakonzentrationen lagen im Bereich von 1.8 bis 3.7 µM und wurden 30-60 min nach der oralen Applikation erhalten. Die ermittelte Bioverfügbarkeit des Dabigatran-Amidoxim-Bernsteinsäureesters (**1**) unterscheidet sich nicht signifikant von den Ergebnissen, die nach oraler Applikation des Dabigatran-Etexilates erhalten wurden. Mit der Entwicklung des Dabigatran-Amidoxim-Bernsteinsäureesters (**1**) ist es somit gelungen, ein dem Dabigatran-Etexilat hinsichtlich Bioverfügbarkeit vergleichbares Prodrug zu entwickeln.

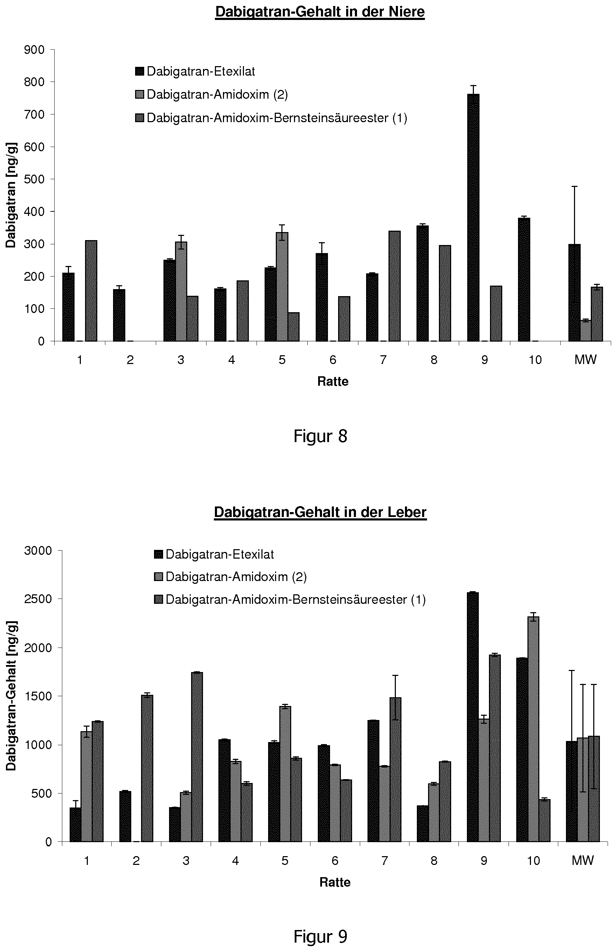

Die Analyse der Organproben (Niere und Leber) zeigte, dass nach oraler Applikation des Dabigatran-Amidoxim-Bernsteinsäureester (**1**) geringe Menge an Dabigatran sowohl in Leber als auch in der Niere zu detektieren sind (Abbildungen 8 und 9).

Bei den neu entwickelten Prodrugs handelt es sich um oral bioverfügbare Prodrugs des Dabigatrans. Durch die Überführung des Dabigatrans in die erfindungsgemäßen Prodrugs konnten wichtige Stoffeigenschaften erheblich optimiert werden. Besonders hervorzuheben ist hier die drastisch verbesserte Löslichkeit des Dabigatran-Amidoxim-Bernsteinsäureesters (**1**), die zu verschiedenen Vorteilen bei der Herstellung und der Anwendung des Arzneimittels führen. So kann durch die verbesserte Löslichkeit auf aufwendige galenische und kostenintensive Formulierungen verzichtet werden. Derzeit wird das Dabigatran-Etexilat als Kapsel mit Weinsäure-haltigen Pellets vermarket (Pradaxa®).⁶ Durch Verwendung des Dabigatran-Amidoxim-Bernsteinsäureesters (**1**) kann auf solch technisch anspruchsvolle Verfahren verzichtet werden. Zudem kann die Applikation und damit die Compliance der Patienten optimiert werden indem nur noch 1 Kapsel/Tablette anstelle der jetzt üblichen 2 Kapseln geschluckt werden müssen.

Die Verbindung besitzt mit Ausnahme des sauren pH-Bereichs eine gute chemische Stabilität. Die ausgeprägte Hydrolyse im sauren Milieu bedingt, dass das Prodrug bei der oralen Applikation als magensaft-resistente Formulierung verabreicht werden sollte, um eine vorzeitige Hydrolyse im Magen auszuschließen.

In den *in vitro* Bioaktivierungs-Assays konnte eine rasche und umfangreiche Aktivierung des Prodrugs zum Dabigatran nachgewiesen werden. Die Aktivierung verläuft unabhängig von Cytochrom P450 Enzymen und birgt somit nicht das Risiko von Interaktionen.^{10,11}

In den abschließend durchgeführten Tierstudien konnte die gute orale Bioverfügbarkeit auch experimentell belegt werden. Die orale Bioverfügbarkeit von 5.5% ± 1.7% unterscheidet sich dabei nicht signifikant von der Referenzverbindung Dabigatran-Etexilat.

Zusammengefasst handelt es sich bei den Dabigatran-Amidoxim-DicarbonsäureDerivaten um ausgezeichnete Prodrugs, die über exzellente physikochemische Parameter verfügen und eine gute orale Bioverfügbarkeit besitzen. Alle untersuchten Eigenschaften vergleichend sind die erfindungsgemäßen Dabigatran-Prodrugs dem Dabigatran-Etexilat deutlich überlegen.

Die beschriebene Erfindung wird durch die beigefügten Figuren noch näher erläutert.
Figur 1: Syntheseschema des erfindungsgemäßen Dabigatran-Prodrugs
Figur 2: Stabilität von Dabigatran-Amidoxim-Bernsteinsäureester (1) bei verschiedenen pH-Werten.
Figur 3: Stabilität von Dabigatran-Amidoxim-Bernsteinsäureester (1) in murinem und humanem Plasma.
Figur 4: Plasmaspiegel des Dabigatrans nach oraler Applikation des Dabigatran-Amidoxim-Bernsteinsäureesters (**1**) (50 mg/kg). Dargestellt sind die Plasmakonzentrationen bei allen untersuchten Ratten (n=10).
Figur 5: Plasmaspiegel des Dabigatrans nach oraler Applikation des Dabigatran-Amidoxim-Bernsteinsäureesters (**1**) (50 mg/kg). Dargestellt sind die Mittelwerte der Plasmakonzentrationen von Dabigatran bei allen untersuchten Ratten (n=10).
Figur 6: Plasmaspiegel des Dabigatrans nach intravenöser (10 mg/kg; n=20) und oraler Applikation des Dabigatran (50 mg/kg, n=3) und oraler Applikation verschiedener Dabigatran-Prodrugs (50 mg/kg, n=10). Dargestellt sind die Mittelwerte der Plasmakonzentrationen von Dabigatran in allen untersuchten Ratten.
Figur 7: Übersicht über die Plasmaspiegel des Dabigatrans nach oraler Applikation verschiedener Dabigatran-Prodrugs (50 mg/kg). Dargestellt sind die Mittelwerte der Plasmakonzentrationen.
Figur 8: Übersicht über den Gehalt [ng/g] an Dabigatran in der Niere nach oraler Applikation der verschiedenen Prodrugs (50 mg/kg). Dargestellt sind die Gehalter der Nieren aller untersuchten Ratten.
Figur 9: Übersicht über den Gehalt [ng/g] an Dabigatran in der Leber nach oraler Applikation der verschiedenen Prodrugs (50 mg/kg). Dargestellt sind die Gehalter der Leber aller untersuchten Ratten.

### Material und Methoden: Ausführungsbeispiele

### Synthesen

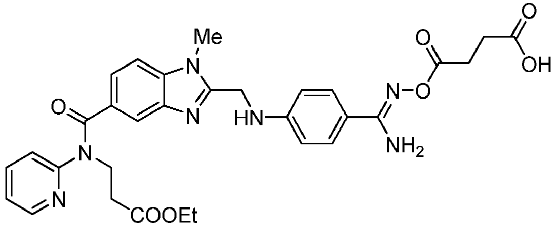

### Ethyl-3-({2-[(4-(N'-(3-carboxypropanoyloxy)amidino)phenylamino)methyl]-1-methyl-1H-benzimid-azol-5-carbonyl}-pyridin-2-yl)propionat (1). Dabigatran-Amidoxim-Bernsteinsäureester (1)

Dabigatran Amidoxim 2 (100 mg, 0.194 mmol) wurden in ca. 8 ml getrocknetem MeCN unter Argon Atmosphäre suspendiert. Bernsteinsäure-Aanhydrid (20.38 mg, 0.204 mmol) wurde zugegeben und die Mischung bei ca. 70 °C für 4 h gerührt (Ölbad auf 80°C eingestellt). Anschließend wurde der Kolben mit Eis gekühlt und ca. 10 ml Diethylether (Et₂O) zugefügt. Der Niederschlag wurde filtriert und sorgfältig mit Et₂O gewaschen.
Ausbeute: 95 mg (80%)
¹H NMR (DMSO-*d₆*):
   δ/ppm (TMS) =1.13 (t, ³*J* = 7.1 Hz, 3H), 2.53, 2.66, 2.69 (3 × t, 6H), 3.77 (s, 3H), 3.98 (q, ³*J* = 7.1 Hz, 2H), 4.23 (br t, 2H), 4.55 (m_{c}, 2H), 6.44 (br s, 2H), 6.62 (br t, 1H), 6.75 (br d, ³*J*= 8.5 Hz, 2H), 6.88 (m_{c}, 1H), 7.13 (m_{c}, 2H), 7.39 (br d, ³*J* = 8.4 Hz, 1H), 7.47 (m_{c}, 3H), 7.54 (br t, 1H), 8.39 (m, 1H), 12.22 (br s, 1H)
¹³C-NMR (DMSO-*d*₆):
   δ/ppm (TMS) = 13.9 (OCH₂*C*H₃), 28.0 (CH₂), 28.8 (CH₂), 29.8 (NCH₃), 33.0 (CH₂), 40.1, 44.3 (2 × CH₂), 60.0 (O*C*H₂CH₃), 109.4 (ArCH), 111.6 (2 × ArCH), 118.9 (ArC), 119.5 (ArCH), 121.2 (ArCH), 122.0 (ArCH), 122.7 (ArCH), 127.5 (2 × ArCH), 129.3 (ArC), 137.2 (ArC), 137.8 (ArCH), 140.8 (ArC), 148.6 (ArCH), 150.0 (ArC), 153.9 (ArC=N), 156.0 (ArC=N), 156.6 (C=NO), 170.3 (CON), 171.0 (2 × COOR), 173.6 (COOH)
HRMS (ESI) m/z:
   Berechnet C₃₁H₃₃N₇O₇ [M + H]⁺: 616.25142; Gefunden 616.25193
Elementar-Analyse C₃₁H₃₃N₇O₇ (molekulare Masse 615.65):
   Berechnet: C 60.48, H 5.40, N 15.93; Gefunden: C 60.16, H 5.24, N 15.87

### Charakterisierung der Dabigatran-Prodrugs

### Stabilitätsuntersuchungen des Dabigatran-Amidoxim-Bernsteinsäureesters (1)

Für die Stabilitätsuntersuchungen wurden eine 0.2 mM Lösung des Dabigatran-Amidoxim-Bernsteinsäureesters (**1**) in 50 mM Kaliumphosphat-Puffer hergestellt. Die Untersuchung erfolgte bei den pH-Werten 2.0, 4.0, 6.3, 7.4 und 9.0. Alle 30 min wurden über einen Zeitraum von 360 min eine Probe gezogen und umgehend per HPLC analysiert.

Weitere Untersuchungen wurden mit humanem und murinem Plasma durchgeführt. Es wurden 900 µl des Plasmas mit 100 µl einer 2 mM Lösung Dabigatran-Amidoxim-Bernsteinsäureesters (**1**) versetzt. Somit betrug die Endkonzentration an Dabigatran-Amidoxim-Bernsteinsäureesters (**1**) 0.2 mM. Die Proben wurden bei 37°C im Wasserschüttelbad inkubiert und nach 0, 15, 30, 45, 60, 90, 120 und 150 min Proben entnommen. Dazu wurden jeweils 100 µl entnommen und mit 100 µl Acetonitril versetzt. Die Proben wurden geschüttelt, für 5 min zentrifugiert und der Überstand mit der HPLC vermessen.

Die Ergebnisse sind in den Abbildungen 2 und 3 dargestellt.

### Löslichkeit von Dabigatran-Amidoxim-Bernsteinsäureester (1)

Eine in 150 µl unlösliche Menge des Dabigatran-Amidoxim-Bernsteinsäureesters (**1**) wurde in 50 mM Phosphatpuffer (pH 6.3, pH 7.4 bzw. pH 9.0) gelöst und für 10 min geschüttelt. Eine Bestimmung der Löslichkeit bei den pH-Werten 4.0 und 2.0 wurde aufgrund der raschen Hydrolyse des Succinylesters bei sauren pH-Werten nicht durchgeführt. Zum Einstellen des pH-Wertes wurde 3 N HCl bzw. 10%ige KOH verwendet. Nach Ablauf der 10 min wurde der ungelöste Teil abzentrifugiert (13,000 RPM, 10 min) und die Proben sofort per HPLC vermessen. Die Auswertung der Löslichkeit erfolgte über eine Kalibrierung von Dabigatran-Amidoxim-Bernsteinsäureester (**1**) (Tabelle 1).

Als Vergleich wurden das Dabigatran-Etexilat und das Dabigatran-Amidoxim (**2**) untersucht, um die Löslichkeit im Vergleich bereits beschriebener Derivate besser beurteilen zu können. Die Bestimmung der Löslichkeiten wurde analog der für Verbindung (**1**) beschriebenen Methode durchgeführt.

**Tabelle 1: Löslichkeit des Dabigatran-Amidoxim-Bernsteinsäureester (1) und anderer Dabigatran-Prodrugs bei unterschiedlichen pH-Werten**

| **Dabigatran-Prodrug** | **Löslichkeit [µM]** | | |
|---|---|---|---|
| | **pH 6.3** | **pH 7.4** | **pH 9.0** |
| **Dabigatran-Amidoxim-Bernsteinsäureester (1)** | 630 ± 290 µM | 4620 ± 830 µM | 8160 ± 440 µM |
| **Dabigatran-Amidoxim (2)** | 145 ± 16 µM | 119 ± 5 µM | 111 ± 8 µM |
| **Dabigatran-Etexilat** | 3.6 ± 2.0 µM | 0.6 ± 0.4 µM | 0.4 ± 0.1 µM |

### Bestimmung der Proteinbindung des Dabigatran-Amidoxim-Bernsteinsäureester (1)

Die Bestimmung der Plasmaprotein-Bindung wurde in drei verschiedenen Konzentrationen (10, 25 und 50 µM) durchgeführt. Als Proteinlösungen wurde eine 4%ige Albumin-Lösung verwendet. Es wurden jeweils 50 µl einer 10fach konzentrierten Substanz-Lösung in 450 µl der Proteinlösung pipettiert. Die Inkubation erfolgte über 15 min im Schüttelwasserbad bei 37°C. Im Anschluss wurden die Proben in Ultrafiltrationseinheiten (Vivaspin 500, 10 kDa cut off) überführt und für 15 min bei 10.000 RPM zentrifugiert. Das Filtrat wurde per HPLC analysiert. Zusätzlich wurde für jede Konzentration eine Kontrolle durchgeführt, die nicht mit Protein versetzt und nicht zentrifugiert wurde. Eine weitere Kontrolle ohne Proteinzusatz, die jedoch durch die Filtrationseinheit abzentrifugiert wurde, diente der Validierung der Methodik.

Die Analyse der Probe ermittelte eine Proteinbindung von 21.8 ± 5.3% für den Dabigatran-Amidoxim-Bernsteinsäureester (**1**). Analoge Untersuchungen für das Dabigatran-Amidoxim (**2**) lieferten Werte von 31.2 ± 1.3%.

### Untersuchung der Bioaktivierung des Dabigatran-Amidoxim-Bernsteinsäureesters (1)

### Ermittlung der Aktivierung der Prodrugs mit verschiedenen subzellulären Enzymsystemen

Die Aktivierung des Prodrugs wurde *in vitro* mittels subzellulärer Enzympräparationen bestimmt. Als Enzympräparationen wurden 9000xg Überstände, Mikrosomen und Mitochondrien aus porcinen Leber- und Nierengeweben verwendet. Die Inkubationsansätze setzten sich aus 500 µM Prodrug, 1 mM NADH, 1 U Esterase und 0.3 mg Enzympräparation, gelöst in 250 µl 100 mM Phosphatpuffer pH 6.3, zusammen. Die Inkubation erfolgte über 30 min bei 37°C im Schüttelwasserbad. Beendet wurde die Inkubation durch Zugabe von 250 µl Methanol. Anschließend wurden die Proben 20 min geschüttelt und das ausgefällte Protein bei 10.000 RPM für 15 min abzentrifugiert. Der Überstand wurde mit Hilfe der HPLC vermessen. Die ermittelten Umsetzungsraten sind in Tabelle 2 aufgeführt.

**Tabelle 2: Aktivierung des Dabigatran-Amidoxim-Bernsteinsäureesters (1) in die Wirkform mit subzellularen Enzympräparationen, SL = Scheineleber, SN = Schweineniere, 9000g = 9000g Überstand, MS = Mikrosomen, Mt = Mitochondrien**

| **Enzymquelle** | **Dabigatran [nmol*min⁻¹*mg⁻¹]** | |
|---|---|---|
| **SN 9000g** | 7.1 | ± 0.9 |
| **SN Ms** | 13.6 | ± 1.1 |
| **SL 9000g** | 8.3 | ± 0.5 |
| **SL Ms** | 18.2 | ± 0.5 |
| **SL Mt** | 15.9 | ± 0.9 |

### HPLC-Analytiken:

Folgende HPLC-Analytiken wurden zur Auswertung verwendet:

### Nachweis von Succinyl-Dabigatran:

| | | | |
|---|---|---|---|
| HPLC-System | Waters Autosampler 717plus, Waters 600 Controller, Waters 600 Pump, Waters 2487 Dual λ Absorbance Detector und EZChrom Elite Client/Server Aufnahme- und Auswertesoftware (Version 2.8.3) | | |
| Stationäre Phase | LiChroCart, LiChrospher 60 RP-select B (VDS Optilab, Länge 125*4 mm, Partikelgröße 5 µm) mit Vorsäule 4*4 mm (Merck) | | |
| Mobile Phase | A | 50% | Methanol |
| | B | 50% | Aqua bidest mit 0.1% TFA |
| | | | 20 mM K₂HPO₄ pH 6.5 |
| Detektion | 293 nm | | |
| Flussrate | 1.0 ml/min | | |
| Laufzeit | 7.5 min | | |
| Injektionsvolumen | 15 µl | | |
| Retentionszeit | Dabigatran-Amidoxim-Bernsteinsäureester (**1**): min | | 2.1 ± 0.1 |
| | Dabigatran Amidoxim (**2**): | | 3.8 ± 0.1 min |
| Nachweis von Dabigatran: | | | |
| HPLC-System | Waters Autosampler 717plus, Waters 600 Controller, Waters 600 Pump, Waters 2487 Dual λ Absorbance Detector und EZChrom Elite Client/Server Aufnahme- und Auswertesoftware (Version 2.8.3) | | |
| Stationäre Phase | LiChroCart, LiChrospher 60 RP-select B (VDS Optilab, Länge 125*4 mm, Partikelgröße 5 µm) mit Vorsäule 4*4 mm (Merck) | | |
| Mobile Phase | A | 30% | Methanol |
| | B | 70% | Aqua bidest mit 0.1% TFA |
| | | | 20 mM K₂HPO₄ pH 4.3 |
| Detektion | 293 nm | | |
| Flussrate | 1.0 ml/min | | |
| Laufzeit | 7.5 min | | |
| Injektionsvolumen | 20 µl | | |
| Retentionszeit | Dabigatran Amidoxim (**2**): | | 4.1 ± 0.1 min |
| | Dabigatran: | | 4.5 ± 0.1 min |

### Orale Bioverfügbarkeit (Tierstudie)

Dabigatran wurde 20 Ratten in einer Konzentration von 10 mg/kg intravenös verabreicht. Der Dabigatran-Amidoxim-Bernsteinsäureester (**1**), Dabigatran-Amidoxim (**2**) und Dabigatran-Etexilat wurden je 10 Ratten in einer Konzentration von 50 mg/kg als Suspension mit Arabischem Gummi (10% m/V) per Schlundsonde appliziert. Zur Herstellung der Suspension wurde beim Dabigatran-Amidoxim-Bernsteinsäureester (**1**) 100 mM Kaliumphosphat-Puffer pH 9.0 verwendet, um eine vorzeitige Abspaltung des Succinylesters im sauren Milieu des Magens zu verhindern. Zusätzlich wurde 3 Ratten Dabigatran in einer Dosierung von 50 mg/kg per Schlundsonde appliziert, um die orale Bioverfügbarkeit der Wirkform selbst zu bestimmen.

Nach i.v.-Gabe wurden Plasmaproben nach 5, 10, 25, 50, 100, 200 und 400 min abgenommen bzw. nach oraler Applikation nach 30, 60, 90, 120, 240, 360 und 480 min. Hierzu wurden 300 µl Vollblut mit einer Insulinspritze entnommen und in EDTA-beschichtete Microvetten CB 300 (Sarstedt, Nümbrecht) überführt. Nach jeder Abnahme wurde mit 100 µl 0.9%-iger Kochsalzlösung bzw. im Abstand von 60 min mit Heparin-Lösung (250 I.E./ml) gespült. Die Blutprobe wurde kurz geschüttelt und bis zur Zentrifugation (4°C; 14000 U/min; 10 min) auf Eis gestellt. Die weitere Lagerung der Proben erfolgte bei -80°C.

Die Tötung erfolgte durch Dekapitation 8 Stunden nach Medikamentengabe mit einer Guillotine. Im Anschluss wurden die Organe entnommen. Alle Organe wurden gesäubert und in mit Trockeneis gekühltem 2-Methylbutan gefroren. Es wurden Leber, Niere, Lunge, Milz, Herz und Gehirn entnommen.

### Probenaufarbeitung

### 1. Plasmaproben:

Die Plasmaproben wurden bei Raumtemperatur aufgetaut. Es wurden jeweils 5 µl 1 N HCl vorgelegt und 55 µl der Plasmaproben zupipettiert. Anschließend wurden die Proben für 45 min geschüttelt, um vorhandene Glukuronide zu spalten. Die Plasmaproteine wurden im Anschluss mit 55 µl Methanol ausgefällt und für weitere 30 min geschüttelt. Die Proben wurden für 15 min bei 10.000 RPM zentrifugiert und der Überstand in HPLC-Vials überführt. Jeweils 10 µl wurden für die Bestimmungen mittels HPLC verwendet.

Zur quantitativen Auswertung der Plasmaproben wurden Kalibrierungen und Analysen zur Wiederfindung des Dabigatrans in Phosphatpuffer pH 7.4 bzw in murinem Plasma durchgeführt.

### 2. Organproben:

Die Organe wurden bei Raumtemperatur aufgetaut und gewogen. Abhängig vom jeweiligen Organ wurden unterschiedliche Mengen der Gewebe aufgearbeitet. Bei den Leberproben wurden ca. 1000 mg verwendet; bei den Nierenproben ca. 500 mg. Leber und Niere wurden untersucht, da beide Organe an der Aktivierung der Prodrugs beteiligt sind und in ihnen aus diesem Grund erhöhte Konzentrationen an Dabigatran auftreten können. Andere Organe spielen für die Bioaktivierung keine Rolle und wurde daher nicht untersucht.

Die Organproben (Leber und Niere) wurden mit Hilfe eines Potters zerkleinert. Dazu wurden die abgewogenen Gewebe mit je 1 ml Aqua bidest für 5 min zerkleinert. Anschließend wurde mit jeweils 1 ml Aqua bidest das Potter-Gefäß ausgespült. Die Proben wurden in Reaktionsgefäße überführt und das gleiche Volumen Acetonitril zugesetzt, um Proteine auszufällen. Die Proben wurden 45 min geschüttelt und anschließend für 15 min bei 12,000 RPM zentrifugiert. Der Überstand wurde in Glasflaschen überführt und unter Druckluft eingeengt. Der Rückstand wurde mit 500 µl Acetonitril gewaschen, erneut zentrifugiert und der Überstand den restlichen Proben zugesetzt. Der Rückstand wurde verworfen.

Nach dem Einengen unter Druckluft wurden die Proben über Nacht gefriergetrocknet.

Das Anlösen der Proben erfolgt mit 400 µl eines Gemisches aus Methanol/Aqua bidest (50/50). Die Proben wurden für 1.5 Stunden bei Raumtemperatur geschüttelt und anschließend der Rückstand abzentrifugiert (15.000 RPM, 15 min). Die Konzentration von Dabigatran wurde aus dem Überstand mittels HPLC bestimmt.

Auf eine Aufarbeitung der Organproben nach oraler Applikation der Wirkform wurde verzichtet, da die Applikation der Wirkform Dabigatran lediglich der Bestimmung der Bioverfügbarkeit dient.

### Ergebnisse der Tierstudie

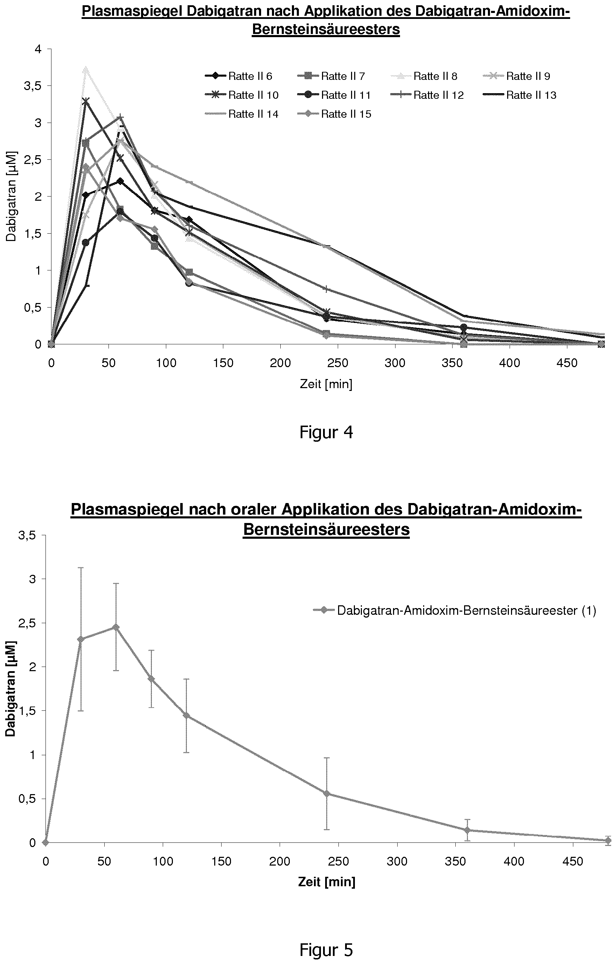

Die Analyse der Plasmaproben nach oraler Applikation des Dabigatran-Amidoxim-Bernsteinsäureesters (**1**) lieferte detektierbare Plasmaspiegel über den gesamten untersuchten Zeitraum von 480 min. Die erhaltenen Plasmapiegel sind in den Abbildungen 4 und 5 dargestellt.

Bei der Analyse der Plasmaproben konnte nur die Wirkform, das Dabigatran, detektiert werden. Das Prodrug selbst war im Plasma nicht nachweisbar, was für eine sehr gute Aktivierung des Prodrugs spricht. Nach oraler Applikation des Dabigatran-Amidoxim-Bernsteinsäureesters (**1**) konnten maximale Plasmakonzentrationen zwischen 1.8 und 3.7 µM bestimmt werden, die 30-60 min nach der oralen Applikation erreicht wurden.

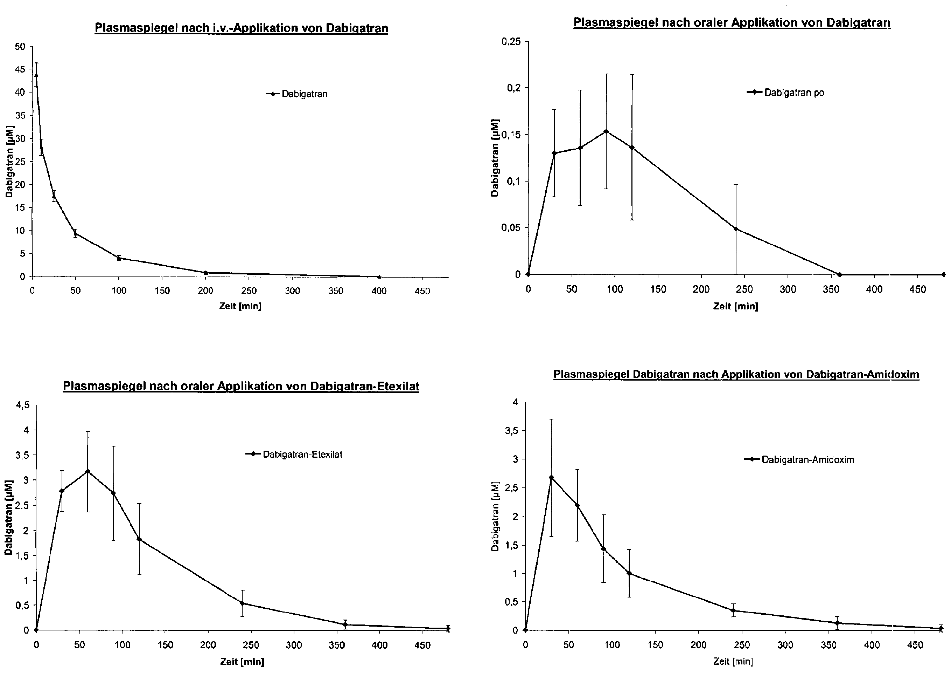

Die Analyse der Plasmaproben nach intravenöser Applikation des Dabigatrans lieferte detektierbare Plasmaspiegel über einen Zeitraum von 400 min (Abbildung 6) und wird zur Berechnung der oralen Bioverfügbarkeit verwendet.

Nach Applikation der beiden Referenz-Prodrugs (Dabigatran-Amidoxim (**2**) und Dabigatran-Etexilat) konnten diese über den untersuchten Zeitraum von 480 min detektiert werden. Die erhaltenen Plasmapiegel sind in Abbildung 6 dargestellt. Bei der Analyse der Plasmaproben konnte jeweils nur die Wirkform, das Dabigatran, detektiert werden. Die Prodrugs selbst waren im Plasma nicht nachweisbar. Nach oraler Applikation des Dabigatran-Etexilates konnten maximale Plasmakonzentrationen zwischen 2.3 und 4.5 µM bestimmt werden, die 30-90 min nach der oralen Applikation erreicht wurden. Nach oraler Applikation des Dabigatran-Amidoxims (**2**) wurden maximale Plasmakonzentrationen zwischen 1.7 und 5.5 µM bestimmt werden, die 30-60 min nach der oralen Applikation erreicht wurden.

### Zusammenfassung und Vergleich der drei Dabigatran-Prodrugs (Abbildung 7):

Ein Vergleich der Ergebnisse der durchgeführten *in vivo*-Studien mit den verschiedenen Prodrugs (Dabigatran-Amidoxim (**2**), Dabigatran-Etexilat und Dabigatran-Amidoxim-Bernsteinsäureester (**1**)) zeigt, dass die höchsten Plasmakonzentrationen nach der Applikation des Dabigatran-Etexilates (7.2% ± 2.0%) bestimmt werden konnten, gefolgt vom Dabigatran-Amidoxim-Bernsteinsäureester (**1**) und Dabigatran-Amidoxim (**2**). Die in der von uns durchgeführten *in vivo* Studie ermittelte Bioverfügbarkeit für das Dabigatran-Etexilat deckt sich somit mit den in der Literatur beschriebenen Daten (5-8%) für das Etexilat.⁶ Die Bioverfügbarkeit des Dabigatran-Amidoxim-Bernsteinsäureesters (**1**) wurde mit 5.5% ± 1.7% ermittelt (Tabelle 3) und unterscheidet sich nicht signifikant von den Ergebnissen, die nach oraler Applikation des Dabigatran-Etexilates erhalten wurden. Mit der Entwicklung des Dabigatran-Amidoxim-Bernsteinsäureesters (**1**) ist es somit gelungen, ein dem Dabigatran-Etexilat vergleichbares Prodrug hinsichtlich der Bioverfügbarkeit zu entwickeln.

### Bioverfügbarkeit der Dabigatran-Derivate:

Die Bioverfügbarkeit der verschiedenen Dabigatran-Prodrugs wurde über die Plasma-Konzentrationen mit Hilfe des Programms PK Solutions 2.0™ berechnet. Des Weiteren wurden die Plasmahalbwertszeit t_{1/2}, der Zeitpunkt der maximalen Plasmaspiegel tₘₐₓ, sowie die maximale Plasmakonzentration cₘₐₓ berechnet. Die erhaltenen Daten sind in Tabelle 3 dargestellt.

**Tabelle 3: Pharmakokinetische Parameter der Dabigatran Derivate**

| | **tₘₐₓ [min]** | **cₘₐₓ [µM]** | **t_{1/2} [min]** | **Bioverfügbar keit [%]** |
|---|---|---|---|---|
| Dabigatran-Amidoxim-Bernsteinsäureester | 48.0 ± 15.5 + | 2.77 ± 0.55 * | 69.3 ± 30.4 ⁺ | 5.5 ± 1.7 ^{+,#} |
| Dabigatran-Amidoxim (**2**) | 36.0 ± 12.6 * | 2.76 ± 1.06 * | 108.1 ± 56.2 ⁺ | 4.1 ± 1.4 * |
| Dabigatran Etexilat | 57.0 ± 22.1 | 3.48 ± 0.64 | 87.7 ± 27.5 | 7.2 ± 2.0 |
| Dabigatran | 105.0 ± 21.2 * | 0.24 ± 0.13 * | 58.0 ± 31.1 ⁺ | 0.3 ± 0.2 * |

| | | | | |
|---|---|---|---|---|
| * p < 0.05 (im Vergleich zu Dabigatran-Etexilat), signifikant ⁺ p > 0.05 (im Vergleich zu Dabigatran-Etexilat), nicht signifikant ^{#} p < 0.05 (im Vergleich zu *N*-OH-Dabigatran), signifikant n.b. = nicht bestimmt (aufgrund sehr starker Schwankungen in den terminalen Plasmaspiegeln) | | | | |

### Auswertung der Organproben:

Die Analyse der aufgearbeiteten Organproben lieferte detektierbare Gehälter an Dabigatran sowohl in Leber als auch in den untersuchten Nieren. In den Lebergeweben wurden vergleichbare Konzentrationen an Dabigatran nach oralen Applikation des Etexilats, des Amidoxims (**2**) und des Succinylesters (**1**) ermittelt. Der Gehalt nach Applikation des Succinylesters war in allen untersuchten Leberproben deutlich geringer (s. Abbildung 9). Die detektierten Gesamtmengen in Leber betrugen durchschnittlich ca. 13 µg bei allen untersuchten Prodrugs. Im Vergleich zu den ermittelten Konzentrationen in den Lebern sind die Gehälter in den Nieren deutlich geringer (s. Abbildung 8). Die in den Geweben nachgewiesen Dabigatran-Gehalte sind allerdings für die Ermittlung der Bioverfügbarkeit unerheblich, da die Bioverfügbarkeit alleine aus den analysierten Plasma-Konzentrationen berechnet wird. Die Dabigatran-Konzentrationen in Leber und Niere dienen lediglich als zusätzliche Informationen, um die neu-entwickelten Prodrugs gut charakterisieren zu können.

### HPLC-Analytiken

Zur Analyse der Organ- und Plasmaproben nach i.v.-Applikation von Dabigatran wurde folgende HPLC-Analytik verwendet:

| | | |
|---|---|---|
| HPLC-System | Waters Alliance™ HPLC-System mit Waters e2695 XC Separations Modul, Waters 2998 Photodiode Array Detector und Empower™ 2 Aufnahme- und Auswertesoftware | |
| Stationäre Phase | LiChroCart, LiChrospher 60 RP-select B (Merck, Länge 125*3 mm, Partikelgröße 5 µm) mit Vorsäule 4*4 mm (Merck) | |
| Mobile Phase | 23% | Methanol |
| | 77% | 20 mM K₂HPO₄ pH 6.5 mit 0.1% TFA |
| Detektion | 210-300 nm (293 nm) | |
| Säulentemperatur | 30°C | |
| Flussrate | 0.7 ml/min | |
| Laufzeit | 9 min | |
| Injektionsvolumen | 10 µl | |
| Retentionszeit | Dabigatran: 5.5 ± 0.2 min | |

Zur Analyse der Organ- und Plasmaproben nach oraler Applikation von Dabigatran-Etexilat, Dabigatran-Amidoxim (**2**), sowie Dabigatran-Amidoxim-Bernsteinsäureester (**1**) wurde folgende HPLC-Analytik verwendet:

| | | | |
|---|---|---|---|
| HPLC-System | Waters Alliance™ HPLC-System mit Waters e2695 XC Separations Modul, Waters 2998 Photodiode Array Detector und Empower™ 2 Aufnahme- und Auswertesoftware | | |
| Stationäre Phase | LiChroCart, LiChrospher 60 RP-select B (Merck, Länge 125*3 mm, Partikelgröße 5 µm) mit Vorsäule 4*4 mm (Merck) | | |
| Mobile Phase | A | Methanol | |
| | B | 20 mM K₂HPO₄ pH 6.5 mit 0.1% TFA | |
| Gradientenprofil | Zeit | A [%] | B [%] |
| | 0 | 77 | 23 |
| | 6 | 77 | 23 |
| | 9 | 50 | 50 |
| | 18 | 50 | 50 |
| | 20 | 77 | 23 |
| | 25 | 77 | 23 |
| Detektion | 210-300 nm (293 nm) | | |
| Säulentemperatur | 30°C | | |
| Flussrate | 0.7 ml/min | | |
| Laufzeit | 25 min | | |
| Injektionsvolumen | 10 µl | | |
| Retentionszeit | Dabigatran: 5.5 ± 0.2 min | | |

### Referenzliste:

1. Holmes, M.; Carroll, C.; Papaioannou, D. Dabigatran etexilate for the prevention of venous thromboembolism in patients undergoing elective hip and knee surgery: a single technology appraisal. Health Technol Assess 2009, 13 Suppl 2, 55-62.
2. Schirmer, S. H.; Baumhakel, M.; Neuberger, H. R.; Hohnloser, S. H.; van Gelder, I. C.; Lip, G. Y.; Bohm, M. Novel anticoagulants for stroke prevention in atrial fibrillation: current clinical evidence and future developments. J Am Coll Cardiol 2010, 56, 2067-76.
3. Weber, R.; Diener, H. C.; Weimar, C. Prevention of cardioembolic stroke in patients with atrial fibrillation. Expert Rev Cardiovasc Ther 2010, 8, 1405-15.
4. Weitz, J. I. Potential of new anticoagulants in patients with cancer. Thromb Res 2010, 125 Suppl 2, S30-5.
5. Plumb, J. M.; Clemens, A.; Monz, B. U. Cost effectiveness of venous thromboembolism pharmacological prophylaxis in total hip and knee replacement: a systematic review. Pharmacoeconomics 2010, 28, 781-2; author reply 782-4, 784-5.
6. Ingelheim, B. Fachinformation Pradaxa 110 mg Hartkapseln. 2011**.**
7. Ingelheim, B. Oral zu applizierende Darreichungsform für 3-(2-(4-(hexyloxycarbonylamidino)phenylaminomethyl)-1-methyl-1H-benzimidazole-5-carbonyl)-2-pyridylamino)propionsäure-ethylester und dessen Salze. 2004**.**
8. Clement, B. Reduction of N-hydroxylated compounds: amidoximes (N-hydroxyamidines) as pro-drugs of amidines. Drug Metab Rev 2002, 34, 565-79.
9. Clement, B. R., C.; Hungeling, H. Use of amidoxime carboxylic acid esters and N-hydroxyguanidine carboxylic acid esters for producing prodrugs. 2009**.**
10. Gruenewald, S.; Wahl, B.; Bittner, F.; Hungeling, H.; Kanzow, S.; Kotthaus, J.; Schwering, U.; Mendel, R. R.; Clement, B. The fourth molybdenum containing enzyme mARC: cloning and involvement in the activation of N-hydroxylated prodrugs. J Med Chem 2008, 51, 8173-7.
11. Havemeyer, A.; Bittner, F.; Wollers, S.; Mendel, R.; Kunze, T.; Clement, B. Identification of the missing component in the mitochondrial benzamidoxime prodrug-converting system as a novel molybdenum enzyme. J Biol Chem 2006, 281, 34796-802.
12. Stangier, J.; Rathgen, K.; Stahle, H.; Gansser, D.; Roth, W. The pharmacokinetics, pharmacodynamics and tolerability of dabigatran etexilate, a new oral direct thrombin inhibitor, in healthy male subjects. Br J Clin Pharmacol 2007, 64, 292-303.
13. Rolan, P. E. Plasma protein binding displacement interactions--why are they still regarded as clinically important? Br J Clin Pharmacol 1994, 37, 125-8.

## Patentansprüche

1. Verbindung der Formel 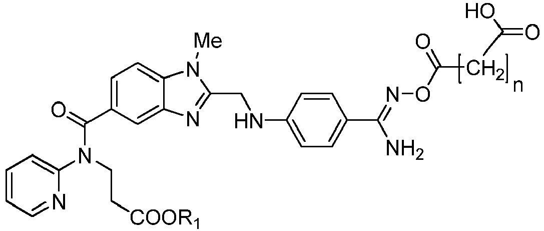 in welcher
R¹ für ein -H, eine verzweigte oder unverzweigte, gesättigte oder ungesättigte, nicht substituierte Kohlenwasserstoffkette mit einer Kettenlänge von 1 bis 12 steht und
n für 1-10 steht.

2. Verbindung gemäß Anspruch 1, wobei n für 2 steht.

3. Verbindung gemäß Anspruch 1 oder 2, wobei R¹ für Ethyl steht.

4. Salze, Solvate und Solvate der Salze der Verbindungen gemäß Anspruch 1 bis 3.

5. Verbindung nach einem der Ansprüche 1 bis 4 zur Verwendung bei der Behandlung und/oder Prophylaxe von Krankheiten.

6. Verbindung nach einem der Ansprüche 1 bis 4 zur Verwendung bei der Behandlung und/oder Prophylaxe von thrombotischen Erkrankungen.

7. Verbindung nach einem der Ansprüche 1 bis 4 zur Verwendung bei der Behandlung und/oder Prophylaxe von thrombotischen Ereignissen, insbesondere der venösen Thromboembolie (VTE).

8. Verbindung nach einem der Ansprüche 1 bis 4 zur Verwendung bei der Behandlung und/oder Prophylaxe von Schlaganfall, Herzinfarkt und/oder Vorhofflimmern und Arrhythmien.

9. Verbindung nach einem der Ansprüche 1 bis 4 zur Verwendung bei der Behandlung und/oder Prophylaxe onkologischen Erkrankungen jeglicher Pathogenese.

10. Arzneimittel umfassend mindestens eine Verbindung nach einem der Ansprüche 1 bis 4 mit einer die Thrombinzeit verlängernden Wirkung, einer thrombinhemmenden Wirkung und/oder einer Hemmwirkung auf verwandte Serinproteasen.

11. Arzneimittel umfassend mindestens eine Verbindung nach einem der Ansprüche 1 bis 4 gegebenenfalls in Kombination mit einem oder mehreren inerten, nicht-toxischen, pharmazeutisch geeigneten Hilfsstoffen.

12. Arzneimittel umfassend mindestens eine Verbindung nach einem der Ansprüche 1 bis 4 in Kombination mit einem oder mehreren weiteren Wirkstoffen.

13. Arzneimittel umfassend mindestens eine Verbindung nach einem der Ansprüche 1 bis 4 zur oralen oder parenteralen Anwendung.

14. Arzneimittel nach einem der Ansprüche 9 bis 12, **dadurch gekennzeichnet, dass** das Arzneimittel magensaftresistent formuliert ist.

15. Verfahren zur Herstellung einer Verbindung gemäß einem der Ansprüche 1 bis 4, **dadurch gekennzeichnet, dass** ein Nitril der Formel (A) 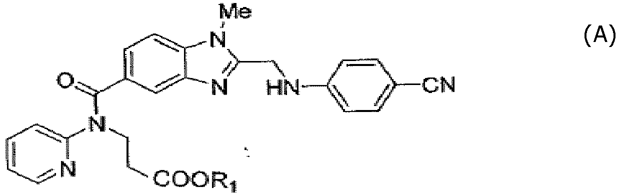 in welcher R¹ für ein -H, eine verzweigte oder unverzweigte, gesättigte oder ungesättigte, nicht substituierte Kohlenwasserstoffkette mit einer Kettenlänge von 1 bis 12 steht
zu einem Amidoxim der Formel (B) umgesetzt wird 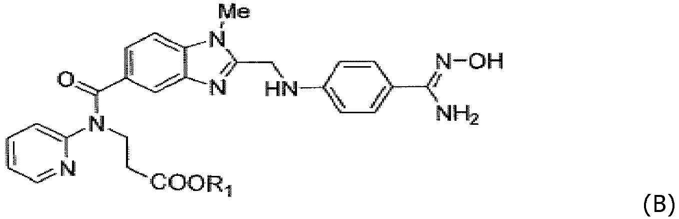 und das so gewonnene Amidoxim durch Reaktion mit einem Dicarbonsäureanhydrid der Formel (C) 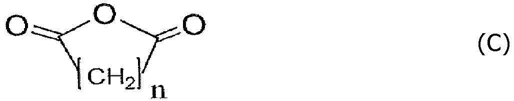 in welcher n für 1-10 steht
in eine Verbindung der Formel 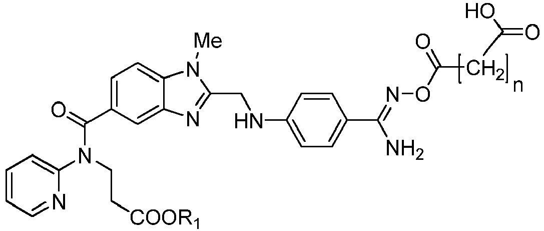 überführt wird.

## Claims

1. Compound of formula 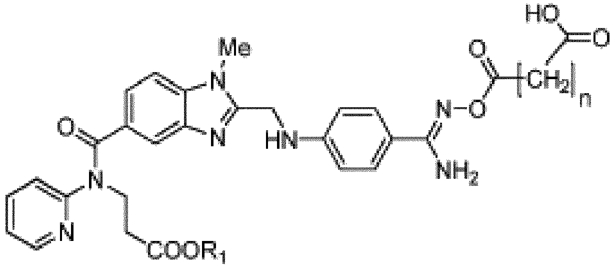 in which
R¹ is an -H, a branched or unbranched, saturated or unsaturated, non-substituted hydrocarbon chain with a chain length of 1 to 12 and
n is 1-10.

2. Compound according to claim 1, wherein n is 2.

3. Compound according to claim 1 or 2, wherein R¹ is ethyl.

4. Salts, solvates and solvates of the salts of the compounds according to claims 1 to 3.

5. Compound according to any one of claims 1 to 4 for use in the treatment and/or prophylaxis of diseases.

6. Compound according to any one of claims 1 to 4 for use in the treatment and/or prophylaxis of thrombotic diseases.

7. Compound according to any one of claims 1 to 4 for use in the treatment and/or prophylaxis of thrombotic events, in particular venous thromboembolism (VTE).

8. Compound according to any one of claims 1 to 4 for use in the treatment and/or prophylaxis of stroke, cardiac infarction and/or atrial fibrillation and arrhythmias.

9. Compound according to any one of claims 1 to 4 for use in the treatment and/or prophylaxis of oncologic diseases of any pathogenesis.

10. Medicine comprising at least one compound according to any one of claims 1 to 4, having a thrombin time-prolonging effect, a thrombin-inhibiting effect and/or an inhibitory effect on related serine proteases.

11. Medicine comprising at least one compound according to any one of claims 1 to 4 optionally in combination with one or several inert, nontoxic, pharmaceutically acceptable excipients.

12. Medicine comprising at least one compound according to any one of claims 1 to 4 in combination with one or several additional active ingredients.

13. Medicine comprising at least one compound according to any one of claims 1 to 4 for oral or parenteral use.

14. Medicine according to any one of claims 9 to 12, **characterized in that** the medicine has been formulated to be resistant to gastric juice.

15. Method for manufacturing a compound according to any one of claims 1 to 4, **characterized in that** a nitrile of formula (A) 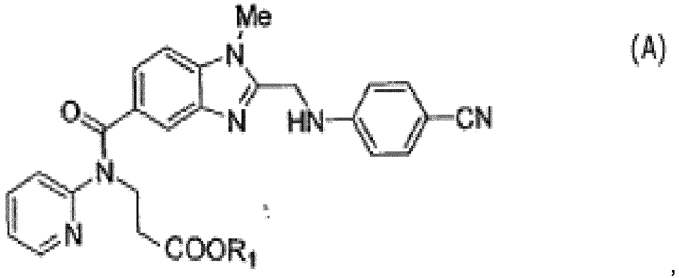 in which R¹ is an -H, a branched or unbranched, saturated or unsaturated, non-substituted hydrocarbon chain with a chain length of 1 to 12,
is converted to an amidoxime of formula (B) 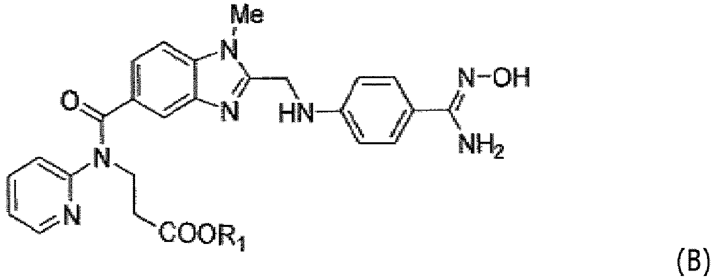 and the amidoxime thus obtained is reacted with a dicarboxylic acid anhydride of formula (C) 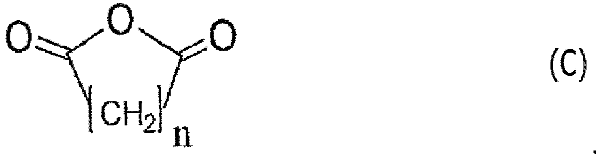 in which n is 1-10
to obtain a compound of formula

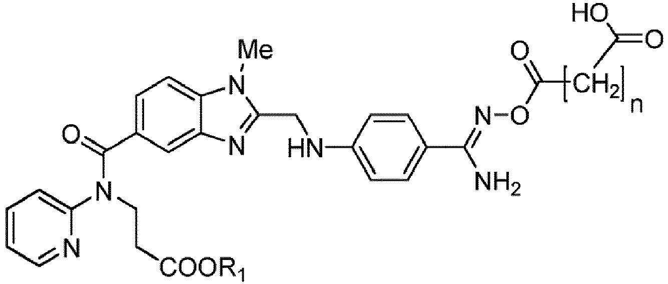

## Revendications

1. Composé selon la formule 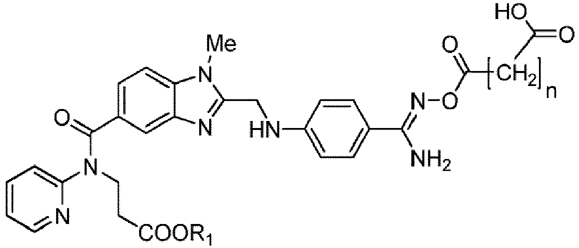 dans laquelle
R¹ représente -H, une chaîne hydrocarbonée non substituée, saturée ou insaturée, ramifiée ou non ramifiée ayant une longueur de chaîne de 1 à 12 et
n représente 1 à 10.

2. Composé selon la revendication 1, dans lequel n représente 2.

3. Composé selon la revendication 1 ou 2, dans lequel R¹ représente un groupe éthyle.

4. Les sels, solvates et solvates des sels des composés selon les revendications 1 à 3.

5. Composé selon l'une quelconque des revendications 1 à 4 pour le traitement et / ou la prophylaxie de maladies.

6. Composé selon l'une quelconque des revendications 1 à 4 pour une utilisation dans le traitement et / ou la prophylaxie des maladies thrombotiques.

7. Composé selon l'une quelconque des revendications 1 à 4 pour une utilisation dans le traitement et / ou la prophylaxie d'événements thrombotiques, en particulier de la thromboembolie veineuse (TEV).

8. Composé selon l'une quelconque des revendications 1 à 4 pour une utilisation dans le traitement et / ou la prophylaxie de l'accident vasculaire cérébral, de l'infarctus du myocarde et / ou de la fibrillation auriculaire et de l'arythmie.

9. Composé selon l'une quelconque des revendications 1 à 4 pour une utilisation dans le traitement et / ou la prophylaxie de maladies oncologiques de toute pathogenèse.

10. Composition pharmaceutique comprenant au moins un composé selon l'une quelconque des revendications 1 à 4 avec un effet prolongeant le temps de thrombine, un effet inhibant la thrombine et / ou un effet inhibiteur sur des protéases à sérine apparentées.

11. Composition pharmaceutique comprenant au moins un composé selon l'une quelconque des revendications 1 à 4 éventuellement en combinaison avec un ou plusieurs excipients inertes, non toxiques, pharmaceutiquement appropriés.

12. Composition pharmaceutique comprenant au moins un composé selon l'une quelconque des revendications 1 à 4 en combinaison avec un ou plusieurs autres ingrédients actifs.

13. Composition pharmaceutique comprenant au moins un composé selon l'une quelconque des revendications 1 à 4 pour l'application orale ou parentérale.

14. Médicament selon l'une des revendications 9 à 12, **caractérisé en ce que** le médicament est formulé entérique.

15. Procédé de préparation d'un composé selon l'une quelconque des revendications 1 à 4, **caractérisé en ce qu'**un nitrile selon la formule (A) 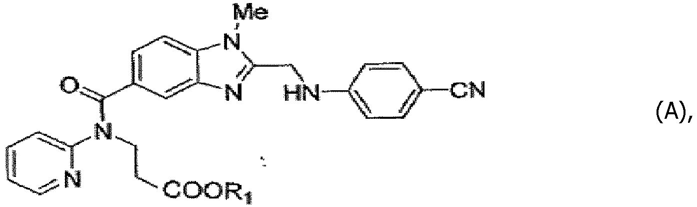 dans laquelle R¹ représente -H, une chaîne hydrocarbonée non substituée, saturée ou insaturée, ramifiée ou non ramifiée ayant une longueur de chaîne de 1 à 12
est mis à réagir à une amidoxime selon la formule (B) 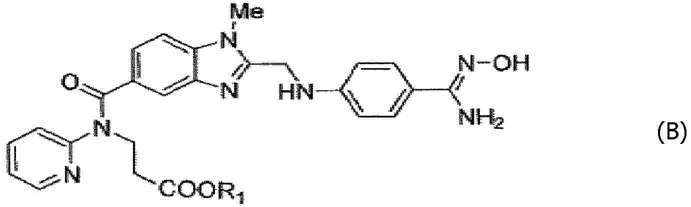 et l'amidoxime ainsi obtenue, par réaction avec un anhydride d'acide dicarboxylique selon la formule (C) 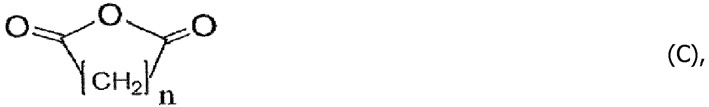 dans laquelle n représente 1 à 10, est transformée dans un composé selon la formule
